Europäisches Patentamt

European Patent Office

Office européen des brevets

(11) Publication number: **0 107 845**
**A2**

(12) # EUROPEAN PATENT APPLICATION

(21) Application number: **83110511.9**

(22) Date of filing: **21.10.83**

(51) Int. Cl.³: **A 61 K 39/02**

(30) Priority: **29.10.82 US 437843**

(43) Date of publication of application:
**09.05.84 Bulletin 84/19**

(84) Designated Contracting States:
**AT BE CH DE FR GB IT LI NL SE**

(71) Applicant: **BACTEX INCORPORATED**
**4307 Bigelow Blvd.**
**Pittsburgh, PA 15213(US)**

(72) Inventor: **Brinton, Charles C.**
**4307 Bigelow Blvd.**
**Pittsburgh Pennsylvania 15213(US)**

(72) Inventor: **Goodnow, Robert A.**
**P.O. Box 3113**
**Omaha Nebraska 68103(US)**

(74) Representative: **Antony, Fritz, Dr. et al,**
**P.O. Box 601 Winkelriedstrasse 35**
**CH-6002 Lucerne(CH)**

(54) **Vaccine for immunizing cattle against infectious bovine keratoconjunctivitis by Moraxella Bovis.**

(57) A vaccine for protecting cattle against Infectious Bovine Keratoconjunctivitis infection by a first group of strains of *Moraxella bovis* (*M. bovis*) contains pili of one or more members of a second group of strains of *M. bovis* which can induce antibodies to crossreact substantially completely with pili from the first group of strains.

EP 0 107 845 A2

Croydon Printing Company Ltd.

- 1 -

<u>Vaccine for immunizing cattle against</u>
<u>Infectious Bovine Keratoconjunctivitis infection</u>
<u>by Moraxella bovis</u>

This invention relates to vaccines for protecting cattle against Infectious Bovine Keratoconjunctivitis infection by <u>Maraxella</u> <u>bovis</u>, and to methods of preparing such vaccines.

The genus <u>Moraxella</u> is a member of the family of the Neisseriaceae. Characteristics of this family include Gram negative plump cells of coccal or coccobacillary shape arranged in pairs, occupation of the mucous membranes of warm blooded animals and existence only as parasites in animals. Bacteria of the <u>Moraxella</u> genus cause conjunctivitis in man and cattle and are also implicated in cases of endocarditis, pneumonia, septicemia, otitis, urethritis and meningitis. They are members of the normal flora of man.

Infectious Bovine Keratoconjunctivitis (IBK) (otherwise known as "pink eye") is a highly contagious disease common in cattle, throughout the world. Organisms of the species <u>Moraxella</u> <u>bovis</u> are considered to be the cause of IBK in cattle and all breeds are susceptible to the disease.

The disease has a low mortality rate but is of considerable economic importance. Economic loss is due to slower growth of calves, reduced milk yields from dairy cows and treatment costs.

The disease is characterized by excessive lachrymal discharge, conjunctivitis, and keratitis and can be described as an inflammation of the conjuctiva and cornea. It first manifests itself by a serous discharge from the eye which later becomes purulent, followed by blepharitis and conjunctivitis. Cloudiness of the center of the cornea can follow in two to four days and an increase in intraocular pressure can cause the cornea to become distended outwards. Impairment of vision occurs at this stage. The disease is associated with ulceration and even rupture of the cornea at a later stage. Pain and photophobia make cattle stand in the shade with their eyes closed. Severe cases of the disease may lead to complete blindness or to deformation of the eye due to the presence of permanent scars. Both unilateral and bilateral infections have been described.

Although IBK occurs throughout the year, it is most prevalent during the summer months when enhancing factors such as fly population and ultraviolet (UV) radiation from the sun are increased. Mechanical injury caused by dust or any other means may allow the microorganism to enter the ocular tissue. Transmission is probably by contact or by mechanical vectors, such as flies.

The Hughes and Pugh research group at The United States Department of Agriculture, National Animal Disease Laboratory in Ames, Iowa has long been interested in M. bovis and particularly in IBK. Their approach to the prevention of IBK was the preparation of vaccines. They tested whole cells, viable [Hughes and Pugh, Am.J.Vet. Res, 32, 879-86(1971), Hughes and Pugh, Am.J.Vet.Res, 33, 2475-79 (1972)], and non-viable (formalin-killed and heat-killed) [Hughes and Pugh, Am.J.Vet.Res, 33, 2475-79, (1972), Hughes et al., Am.J.Vet.Res, 37, 1291-95, (1976)], as well as disrupted cells, [Pugh and Hughes, Can.J.Comp.Med., 40, 60-66 (1976)]. They also reported the purification and use of pili as a vaccine component, and vaccination essentially by the subcutaneous or intramuscular route. The publications, however, did not substantiate the presence, identification or concentration of pili in the vaccine and it is noteworthy that no significant protection was obtained. Pugh and Hughes, Can.J.Comp.Med., 40, 60-66 (1976), Pugh et al, Am.J.Vet.Res., 38, 1519-22 (1977), Pugh et al, Am.J.Vet.Res., 41, 1611-14 (1980).

Another vaccine prepared with a commercially available M. Bovis autogenous bacterin was also unsuccessful, [Arora et al, Am.J.Vet.Res., 37, 803 (1976)]. The vaccine was administered into third eyelids to increase local resistance of the ocular tissue.

In spite of all the above efforts, an effective IBK vaccine has not been achieved.

- 4 -

According to the present invention there is provided a vaccine composition for cattle, capable of raising the antibody level of cattle to a level sufficient to provide protection against infection by one or more strains of M. bovis, said vaccine comprising pili derived from one or more strains of M. bovis capable of inducing antibodies able to cross-react with pili of said first mentioned strain or strains to an extent sufficient to provide protection thereagainst, and a pharmaceutically acceptable carrier.

In one preferred embodiment the vaccine pili is provided by killed or attenuated piliated whole cells, preferably formalinized whole cells. Preferably such piliated whole cells will have been derived from piliated M. bovis by cloning for the piliated phase so as to maximize the amount of pili in relation to the amount of other cellular components.

In another preferred embodiment the killed or attenuated piliated whole cells may be supplemented by pure pili, the purpose being to increase the total pili concentration.

In a most preferred embodiment the vaccine comprises separate pili in the substantial absence of other M. bovis cellular components

It is possible, but least preferred, for the vaccine to comprise separate pili and killed or attenuated non-piliated whole cells.

- 5 -

In essence the vaccine composition of the invention provides protection against a first group of strains of <u>M. bovis</u> in that it comprises pili from at least one member of a second group of strains of piliated <u>M. bovis</u> organisms which can induce antibodies able to <u>cross-react</u> substantially completely with pili from the first group, and the first group of strains may be the same as or different from the second group of strains.

It is in the nature of pili we use that pili from certain strains of <u>M. bovis</u> can induce antibodies capable of reacting against not only pili of the same strains but also against pili of one or more different strains. By judicious selection of the strains from which the vaccine is prepared the protection conferred by the vaccine can extend beyond the homologous vaccine strain to various heterologous strains.

Using known chemical typing methods (in which the chemical identity of each pilus type is established by peptide mapping and amino acid analysis) together with serological examination (using the indirect ELISA method of Voller [Voller et.al., Bull World Health Organisation, 53, 55-65 (1976)] to study the serology of purified M. bovis pili in rabbits) it can be demonstrated that certain strains of M. bovis organisms have the same pilus type. In this respect it is observed that the pilus of each strain of M. bovis may be characterized as having three sets of components, one set being common to all pili of the M. bovis family and capable of inducing antibodies which react with pili of its own strain and cross-react with pili of all other strains of the M. bovis family and has been named the "common antigenic determinant set", the second set is capable of significantly inducing antibodies which will react with pili of its own strain and possibly one or more other strains but not with pili of all other strains, and has been named the "variable antigenic determinant set." The third set is common to pili of all strains of M. bovis but is not involved in antibody induction, and has been named the "common sequence other than the common antigenic set".

This analysis has been applied for example to 16 M. bovis strains with the results shown in the following Table I

0107845

- 7 -

**TABLE I**   Names of M. bovis pilus types according to structural / serological data.

| Strain group | Strain | Pilus type |
|---|---|---|
| I | WSE 64 (21)<br>IBH 68 | Pil Mbo $C_1V_1S$ |
| II | EPP 63 | Pil Mbo $C_1V_2S$ |
| III | 1965 Glenn 5004<br>Him 63 | Pil Mbo $C_1V_3S$ |
| IV | IVI 64<br>MED 72 (4R)<br>MED 72 (19L)<br>WSE 64 (13R) | Pil Mbo $C_1V_4S$ |
| V | MAC 74 (2554R)<br>MAC 74 (2562R) | Pil Mbo $C_1V_5S$ |
| VI | FLA 64<br>ATC 10,900<br>8613 (1) | Pil Mbo $C_1V_6S$ |
| VII | NTN 63 | Pil Mbo $C_1V_7S$ |
| VIII | GIN 63 | Pil Mbo $C_1V_8S$ |

in the above table C = common antigenic determinant set

V = variable antigenic determinant set

S = common sequence other than C.

Pili from twelve of these strains have been examined for serological cross reactivity using the ELISA method with rabbit anti-serum, and the results are set forth in the following Table II.

- 8 -

The cross-reactivity of pilus antibodies can be determined using M. bovis pilus-specific rabbit antiserum as follows:

Obtain pure dialyzed pili by the method described herein and mixed with an equal weight of Freund's Incomplete Adjuvant.

Take New Zealand white rabbits and take blood samples from a lateral ear vein to obtain a pre-immune control serum. Then inject the rabbits subcutaneously using an immunization schedule of the primary injection and two boosters approximately two weeks apart. Use a dosage of 50 to 100 $\mu$g of the vaccine per 2.5 kg of body weight. Two rabbits per strain can be injected and the results averaged. Immune sera can be obtained by cardiac puncture. Serological study of the various antisera reactivities against the various pili can then be carried out by the aforementioned indirect ELISA methods.

The figures given in the following table are for the neutralization factor of pili-induced antibodies in antiserum with pili from heterologous strains of M. bovis. In all cases the neutralization factor of the pili-induced antibodies in antiserum with respect to pili from the homologous strain has been rounded up to 100.

## TABLE II

### NEUTRALIZATION FACTOR OF PILI INDUCED
### ANTIBODY IN ANTISERUM AGAINST PILI

| Group | I | | II | III | IV | | | | V | VI | | |
|---|---|---|---|---|---|---|---|---|---|---|---|---|
| Antiserum / Pilus | WSE 64 (2L) | IBH 68 | EPP 63 | 1965 Glenn 5004 | IVI 64 | MED 72 (4R) | MED 72 (19L) | WSE 64 (13R) | MAC 74 2554 R | FLA 64 | ATC 10,900 | 8613 |
| WSE 64 (2L) | 100 | 70 | 39 | 35 | 20 | 6 | 15 | 13 | 6 | 8 | 4 | 9 |
| IBH 68 | 61 | 100 | 36 | 34 | 24 | 7 | 17 | 9 | 8 | 13 | 4 | 6 |
| EPP 63 | 13 | 30 | 100 | 36 | 16 | 12 | 11 | 12 | 6 | 7 | 4 | 5 |
| 1965 Glenn 5004 | 14 | 36 | 30 | 100 | 23 | 10 | 17 | 10 | 7 | 5 | 3 | 7 |
| IVI 64 | 16 | 40 | 52 | 36 | 100 | 97 | 111 | 87 | 9 | 14 | 5 | 8 |
| MED 72 (4R) | 28 | 40 | 70 | 39 | 91 | 100 | 164 | 79 | 9 | 16 | 7 | 7 |
| MED 72 (19L) | 11 | 40 | 68 | 31 | 100 | 119 | 100 | 84 | 8 | 10 | 7 | 6 |
| WSE 64 (13R) | 15 | 51 | 84 | 41 | 83 | 122 | 102 | 100 | 9 | 17 | 4 | 6 |
| MAC 74 2554R | 16 | 34 | 28 | 31 | 26 | 6 | 17 | 23 | 100 | 10 | 4 | 9 |
| FLA 64 | 14 | 27 | 23 | 32 | 22 | 5 | 15 | 7 | 7 | 100 | 54 | 101 |
| ATC 10,900 | 20 | 25 | 35 | 30 | 21 | 7 | 28 | 14 | 11 | 165 | 100 | 108 |
| 8613 | 16 | 26 | 20 | 33 | 17 | 8 | 18 | 5 | 7 | 81 | 88 | 100 |

0107845

It will be noted from the table that cross-reaction occurs with respect to all strains to a greater or lesser extent; this is because, as explained above, all members of the M. bovis pilus family have a common antigenic determinant set.

The thick outlines surround figures indicating substantially complete cross-reactivity. The strains associated with the pili and anti-serum involved can be seen to correlate with the members of the strain groups in Table 1, that is, the substantially completely cross-reactive strains have the same pilus types.

In practical terms the cross-reactivity results demonstrate that certain strains of M. bovis carry the same types of pilus. In order to protect against infection by any member of a given group containing the same pilus types the vaccine must contain an effective amount of pili from that group, that is pili of the same type but not necessarily of the same strain. It will be appreciated that whilst such vaccine will be apparently offering strain heterologous protection, it in fact will be providing pilus-type homologous protection.

In the design of a vaccine it should preferably be aimed at selecting the antigen or antigens capable of inducing antibodies which confer protection against the broadest range of pathogenic subtypes.

A vaccine containing a few pilus types can provide protection against many M. bovis strains. Accordingly it is possible to design a polyvalent vaccine with pilus from relatively few strains which will provide a broad range of protection against many more strains of the M. bovis family.

Strains of <u>M. bovis</u> can be isolated from the eyes of cattle suffering from IBK.  Less frequently they can be isolated from the eyes of normal cattle.  Various strains may also be obtained from Culture Centres.

Among the strains which can be used are for example:

| Strain | ATCC No. |
| --- | --- |
| EPP 63 | 39218 |
| FLA 64 | 39219 |
| WSE 64 (2L) | 39231 |
| MED 72 (4R) | 39220 |
| IBH 68 (712L) | 39222 |
| MED 72 (19L) | 39223 |
| NTN 63 (8033L) | 39232 |
| Him 63 (5R) | 39233 |
| IVI 64 (58L) | 39224 |
| 1965 GLENN 5004 | 39225 |
| WSE 64 (13R) | 39221 |
| MAC 74 (2554R) | 39228 |
| MAC 74 (2562R) | 39226 |
| ATC 10,900 | 39230 |
| 8613 (1) | 39227 |
| GIN 63 | 39229 |

Preferably the pili is derived from one or more of the following piliated strains of <u>M. bovis</u>

|      |              | ATCC No. |
|------|--------------|----------|
| IBH  | 68           | 39222    |
| EPP  | 63           | 39218    |
| 1965 | Glenn 5004   | 39225    |
| MED  | 72 (19L)     | 39223    |
| MAC  | 74 (2554R)   | 39228    |
| FLA  | 64           | 39219    |
| NTN  | 63           | 39232    |
| GIN  | 63           | 39229    |

and strains having the same pilus type as the above strains.

Preferably the vaccine contains pili of at least six of the strains set forth in the preceding paragraph; or pili which is capable of inducing antibodies substantially completely cross-reactive with pili of at least six of said strains; or pili of some of said strains set forth above <u>and</u> pili capable of inducing antibodies substantially completely cross-reactive with some of the strains set forth above such that the combined pili is capable of inducing antibodies substantially completely reactive with at least six of said strains.

A vaccine in accordance with the invention may be in a form suitable for oral administration, e.g. in capsule form, or parenteral administration e.g. in injectable dosage form for subcutaneous, intradermal or intramuscular injection. Where the vaccine is in injectable form any pharmaceutically acceptable suspending medium may be employed.

A vaccine in accordance with the invention may comprise the pili and/or whole cell material simply in suspension in water, saline or maybe based on a pharmaceutically compatible buffer solution in which the pili is soluble, for example KPB pH=7.0, $\mu$=0.01 as mentioned below. Usually however the vaccine will also contain other conventional vaccine components, such as preservatives, adjuvants and so forth. Formalin is a suitable preservative and may be used in concentrations conventially used in vaccines, preferably in a concentration equivalent to 0.2 to 0.4% v/v of a 37% formalin solution (for example 0.3ml of such formalin solution per 100ml of vaccine composition). A suitable adjuvant is aluminium hydroxide, which maybe utilized as an aluminium hydroxide gel or suspension. This maybe included in the vaccine in an amount such that the $Al_2O_3$ assay of the vaccine shows 0.4 to 0.6% by weight $Al_2O_3$ preferably 0.5% by weight.

A preferred vaccine suspension medium is saline containing formaldehyde.

Preferably an injectable vaccine composition has a concentration of 1 to 5 mg, e.g. 1 to 4 mg preferably 3 to 5 mg of pili per 3ml of suspending medium when pure pili is used; a whole cell vaccine will contain preferably 2 to 6 mg, most preferably 5 mg of cellular meterial (including the pili) per 3 ml of suspending medium.

The weight ratio of pili to whole cell debris is usually approximately 3:2. Thus where a pure pilus vaccine containing a dose of approximately 3 mg is sufficient to provide protection, a whole cell vaccine containing approximately 5 mg of cellular material would be required to give a comparable degree of protection.

An injectable vaccine suspension can suitably be prepared to meet the generally accepted standards of syringeability using well known methods.

It is generally preferred, though not critical, to administer the vaccine composition in more than one dose separated by a predetermined time factor. The time factor can be selected by the veterinary worker to permit the formation of an adequate titre of antibodies in the subject being treated.

It has been found suitable to administer between 5 to 40 micrograms of pili per kilogram of body weight. Dosage has, however, not been found very body weight sensitive as similar dosages are desirable for calves and adult animals.

In broadest terms a process for preparing a vaccine in accordance with the present invention comprises establishing a mixture of pili and/or piliated whole cells of one or more strains of M. bovis with a pharmaceutically acceptable carrier or excipient.

Pili and/or whole cells may be mixed with a pharmaceutically acceptable carrier or, for example pili containing contaminants may be dialyzed against a suitable carrier e.g. water, saline and so forth, until the medium containing the pili is of acceptable purity.

In a preferred process the preparation of a pilus-only vaccine involves treating separated pili to remove undesired salts or any other remaining contaminants, preferably by dialysis using standard methods. For instance the pilus (from one or more M. bovis strains, obtained by the above methods is preferably suspended in the desired medium e.g. water, saline or potassium phosphate buffer (KPB) containing NaCl and formalin, and dispensed within a semi-permeable membrane with the corresponding pure water, saline or KPB solution dispensed on the other side of the membrane. Diffusion of the contaminants through the membrane will eventually result in a pili solution or suspension of desired purity.

The resulting solution, if necessary or desired is mixed with conventional vaccine components such as a preservative e.g. formalin or adjuvants e.g. aluminium hydroxide, (if not already present in the solution) and is dispensed into vials.

If desired the purified pili obtained as described above can be used to supplement a vaccine containing piliated whole cells or even non-piliated cells.

A whole-cell piliated vaccine is preferably prepared from piliated cells grown as described below. After removal from the culture medium the cells can be killed or attenuated by known procedures and the biomass mixed or agitated to reduce particle size.

Preferably the cells are inactivated by the addition of formalin to a suspension of cells. For instance formalin may be added to the surface growth of M. bovis, which has been washed off agar culture medium with trypiticase soy broth, in an amount of 0.3% by volume.

The wet particle size is preferably reduced, before or after such inactivation or attenuation, to the range of 0.01 to 0.2mm.

Any desired additives are added at a suitable stage, generally after any necessary reduction in particle size.

For instance, preservatives and adjuvants may be added, as described above in relation to the pili only vaccine, and if necessary or desired separate pure pili is added to supplement the cell-bound pili.

The whole-cell composition is then dispensed in suitable vials.

Strains of M. bovis can be used which have been isolated from cattle, usually infected parts of cattle suffering from IBK or from culture collections.

On primary isolation from cattle M. bovis produces small, flat, umbonate, firm and dry colonies with a slightly irregular outline and a darkly rimmed edge. These colonies have adhesive characteristics; when touched by an inoculating loop they will stick to it, and they autoagglutinate when suspended in liquids. They are designated "rough" colonies  Such colonies have been described in the literature as having a spreading-corroding character and are generally designated as the "S-C" colony form of M. bovis. S-C colonies are often characterized in that they are β-hemolytic. When grown on agar, they pit the agar surface so that a depression is formed on the agar surface beneath the colonies. A thin layer of growth of variable diameter is very often present around the colonies.

It is found that the organisms of S-C colonies, having the above characteristics, are piliated.

If S-C colonies are repeatedly transferred on artificial media (without care specifically to clone for the S-C colony type) it will eventually be found that the colony type changes so that smooth colonies lacking the auto-agglutinating and adhesive characteristics of the S-C colony type and which do not pit the agar surface are being grown. The smooth colony type is non-corroding or non-spreading or only weakly so: such colonies have been designated in the literature as the "N" colony form.

- 18 -

It is found that organisms of N-colonies, having the above characteristics are non-piliated.

The switch from one colony type to another is due to piliated phase variation, this being a regulatory mechanism affecting the phenotypic expression of piliation. Bacteria which are somatically piliated may, as a rare event but at a high frequency compared to mutation rates, spontaneously switch from its existing phenotypic state (piliated) to the other phenotypic state (non-piliated) and vice-versa. Each form is metastable, that is, the daughter cells retain the same state as the mother cells unless there is a phase change. The phase change is reversible and the rates of change are different in the two directions from piliated to non-piliated and are affected by environmental conditions.

In general the conditions which favour the production of piliated M. bovis organisms are growth in vivo, non-agitated liquid media, minimal media, and high cell concentration.

The conditions favouring growth of non-piliated organisms are growth in vitro, agitated liquid media, rich media and low cell concentrations. Thus, for instance, growth in solid media without repeated cloning and growth in shaking aerated liquid media, could select for the non-piliated phase while still, non-aerated liquid media conditions could select for the piliated phase.

However, even under conditions favouring the piliated phase, a number of cells in the overall population are in the non-piliated phase and vice versa.

Cultures which contain predominantly the piliated phase can be grown by carrying out frequent cloning of colonies of the piliated cells. Cultures of the different colonial morphologies can be identified and the derived SC-colony type can be maintained by continuous cloning, which is usually done by repeatedly transferring the desired colony type: in this way a homogeneous culture can be obtained.

In more detail, M. bovis piliated cells, and separate M. bovis pili are preferably prepared according to the following procedure. The described procedure goes right through to the preparation of separate pili. Piliated whole cells can be obtained by curtailing the procedure at a stage when the whole cells have been harvested.

All the above strains were isolated originally from IBK infected cattle. All are capable of yielding piliated cells or purified pili. In cultivating the above strains both S-C colonies and N colonies may develop under the appropriate conditions. In the cases of EPP 63, FLA 72 (4R), NTN 63 (8033L), and NDL 67 (950L), colonies with one or two other different morphologies may develop. However the S-C colony type described above should be cultivated by cloning, as mentioned above, to obtain the desired piliated cell colonies.

The preferred media for growing and cloning strains of Moraxella bovis have the following compositions and/or are formulated as follows:

1. A trypticase soy agar with 5% sheep blood (BBL) having the composition

| | |
|---|---|
| Trypticase peptone | 15.0g |
| Phytone peptone | 5.0g |
| NaCl | 5.0g |
| Agar | 15.0g |
| Sheep blood, defibrinated | 2.0g |

2. A blood agar is formulated by first preparing, according to the manufacturer's instructions, a tryptose blood agar base (Difco) having the following composition

| | |
|---|---|
| Bacto-Tryptose | 10.0g |
| Bacto-Beef extract | 3.0g |
| NaCl | 5.0g |
| Bacto Agar | 15.0g |

cooling the base down to the temperature range 45-50°C, adding 5% by weight of fresh defibrinated blood and dispensing the mixture into petri-dishes.

3. A Müller-Hinton broth (Difco) is prepared by dissolving in 1 litre of distilled water dehydrated powder mixture of the following composition

| | |
|---|---|
| Beef infusion | 19.7g |
| Casamino-acids technical | 1.2g |
| Bacto-soluble starch | 0.1g |

and then sterilizing for 15 minutes.

4. A medium is prepared from a GC Agar Base Medium (Difco) as follows:

0.5ml of a 0.4% sterile solution of cocarboxylase are added to 100ml of an aqueous solution containing by weight 40% dextrose, 1% L-glutamine and 0.05% ferric nitrate, balance being water. 15ml of this mixture are added to a litre of GC agar base medium from Difco (previously sterilized then cooled down to 45-50°C) having the composition

| | |
|---|---|
| Proteose Peptone No. 3 | 15.0g |
| Corn Starch | 1.0g |
| $K_2HPO_4$ | 4.0g |
| $KH_2PO_4$ | 1.0g |
| NaCl | 5.0g |
| Bacto agar | 10.0g |
| Distilled water | |

- 22 -

5. <u>Müller-Hinton Agar</u>

   a) BBL Catalogue No. 96242

| | |
|---|---|
| Beef extractives | 2.0g |
| Acidicase peptone | 17.5g |
| Starch | 1.5g |
| Agar | 17.0g |
| Distilled water | 1.0 litre |

Prepare according to the manufacturer's instructions, with a final pH of $7.4 \pm 0.2$.

   b) BBL Catalogue No. 11438

| | |
|---|---|
| Beef extractives | 2.0g |
| Acid hydrolysate of Casein | 17.5g |
| Starch | 1.5g |
| Agar | 17.0g |
| Distilled water | 1.0 litre |

Prepare according to the manufacturer's instructions, with a final pH of $7.3 \pm 0.2$

   c) Difo Laboratories Catalogue No. 0.252-01-4

| | | |
|---|---|---|
| Beef infusion | from | 300.0g |
| Bacto-Casaamino acids technical | | 17.5g |
| Starch | | 1.5g |
| Bacto-Agar | | 17.0g |
| Distilled water | | 1.0 litre |

Prepare according to the manufacturer's instructions, with a final pH of $7.3 \pm 0.1$

Incubation can preferably be carried out on a suitable media in a Forma incubator preferably at 35°C with or without 5% $CO_2$ partial pressure and preferably at 80% humidity; or may for example be carried out in a candle jar using wet paper towels (or other absorbent material) saturated with water to provide the appropriate humidity, and at approximately 37°C. Cloning of the piliated phase should preferably be done every 20 to 24 hours to obtain highly homogeneous and stable cultures. Allowing growth to occur for longer periods of time between cloning operations leads to cultures showing reversion from the piliated to non-piliated phase.

Cloning is preferably carried out using Müller-Hinton Agar from BBL catalogue No. 96242: it may also be carried out using GC Agar Base Medium (Difco) supplemented as described above but this gives decreased yields of pili compared with using the first mentioned medium.

Müller-Hinton Agar BBL Catalogue No. 11438 and Difco Catalogue No. 0252-01-4 are both adequate for growth of the microorganism but not for pili production: the Difco medium producing very reduced yields of pili and the Müller-Hinton medium giving no discernible pili product.

Conveniently growth is preferably carried out initially in petri dishes containing a suitable growth medium with e.g. 24 colonies of the desired S-C colony type and incubated preferably as described above in a Forma incubator. After preferably 20 to 24 hours the resulting growth is harvested, preferably with 0.070% aqueous casamino acids to give a suspension of the desired cells in the casamino acids solution: harvesting with e.g. 6ml of the casamino acid solution will yield about 4 to 5ml of cell suspension. The resulting cell suspension is then preferably used to inoculate a further body of suitable growth medium: for example sterile aluminium trays containing approximately 500ml of suitable Müller-Hinton agar medium are conveniently inoculated with 2 to 2.5ml of the cell suspension mentioned above. The resulting medium is then incubated for 20 to 24 hours in a Forma incubator under the same conditions as described above.

The resulting further growth is harvested, preferably with aqueous potassium phosphate buffer solution (KPB) having a pH of 7.0 and an ionic strength of 0.01. Growth from a tray as described above is conveniently harvested with 10ml (per tray) of the buffer solution. The KPB buffer is preferred for this operation as pili are soluble in it, making for convenience in the further processing.

Pili are then separated from the cells. Conveniently this is carried out by shearing which is preferably carried out by blending a suspension of the cells (preferably in KPB buffer) in a high speed mixer at e.g. 10,000rpm using e.g. an Omni mixer. Mixing is preferably carried out for two minutes to cause the pili to be sheared from the cells.

Cell debris are then separated from the pili. This is conveniently done when the continuous phase of the suspension is a liquid in which the pili, but not the cell debris are soluble (e.g. the KPB buffer) and may be
achieved by centrifuging at 16,000xg for one hour.

Pili are then isolated.

In a preferred embodiment this is carried out using the
following operations:

a) pili are salted out from solution in KPB buffer using
   a saturated solution of ammonium sulphate (SAS) which
   is added to the solution of pili in KPB buffer to
   give a final concentration of 10% by volume SAS.
   Under these conditions the pili is salted out,
   aggregating longitudinally to form crystals which
   are visible by dark field microscopy. The solution is
   kept at room temperature for 20 minutes and the pili
   separated by centrifuging for one hour at 16,000xg
   and the supernatant is discarded;

b) the resulting pili pellet is resuspended in half the
   original volume of KPB and is stirred until completely
   solubilized. The pili solution is then clarified by
   centrifuging at 27,000xg for one hour.

c) The pellet is discarded and the supernatant centrifuged at 70,000xg for one hour to remove particulate
   contaminants which cannot be removed by the foregoing
   crystallisation/solublisation operations.

Operation a) and b) and if necessary c) are repeated as
many times as required to achieve the degree of purity
desired; usually three or four such repetitions are sufficient to obtain pili which are pure as determined by sodium dodecyl sulfate polyacrylamide gel electrophoresis (SDS-PAGE), UV light spectroscopy, darkfield-microscopy and electron microscopy.

## EXAMPLE 1

A. Prepare pili crystals from M. bovis EPP 63 by the procedure described above. Dialyze the pili to remove contaminating salts, mainly ammonium sulphate. Dialysis may be against water or KPB buffer pH=7.0, $\mu$=0.01 containing 0.85% by weight NaCl and 0.1% by volume formalin, and is carried out for 24 hours at about 4°C with continuous stirring. The water or KPB buffer outside the membrane containing the pili solution is changed twice during this operation.

Add aluminium hydroxide to the resulting pili solution in an amount corresponding to one quarter the weight of pili therein. Dispense the resulting mixture in injection vials in an amount corresponding to 5 mg pili per vial.

B. A vaccine prepared as above was used in the following experiment to demonstrate the antigenicity of M. bovis EPP 63 pili.

10 calves weighing 112.5 to 135 kg were selected. 5 were to be vaccinated using vaccine as prepared in this Example (test calves) and 5 were to be used as controls (control calves).

The five test calves were each injected, intramuscularly (at a dose of 5 mg of pili) with the above vaccine. Twenty eight days later the injection was repeated (a booster injection).

C. Sera was taken from the test calves i) before the first injection (pre-immune sera), ii) after the first injection, and iii) after the booster injection. Upon testing for antigenicity using the above mentioned ELISA system a progressive increase in neutralization factor was demonstrated in respect of the test calves compared with the control calves as shown in the following table.

Average serum antibody neutralization factor

|  | Pre-immune sera | Post-primary injection sera | Post-booster injection sera |
| --- | --- | --- | --- |
| Controls | 2 | 4 | 5 |
| Vaccinated | 4 | 125 | 448 |

The above values are the averages of those ascertained for all the respective calves, except the "controls" pre-immune sera value which was the average of the values obtained for only four of the control calves.

D. Twelve days after the booster injection, both the test and control calves were challenged each with an $ID_{60}$ of 1.9 x $10^8$ M. bovis EPP 63 piliated cells. Three of the control calves contracted IBK whereas all the test calves were found to be protected.

EXAMPLE 2

Parts A, B and D of Example 1 was repeated but this time challenging calves with $2 \times 10^8$ M. bovis EPP 63 piliated cells. Two of the control calves contracted the disease whereas all the test calves were found to be protected.

EXAMPLE 3

Seven calves similar to those used in the previous Examples are selected. Four calves are used as controls and three as test calves. The three test calves are each vaccinated by injection delivering 1 mg of M. bovis EPP 63 purified pili, followed twenty eight days later by a booster injection, again delivering but 1 mg of the M. bovis EPP 63 purified pili.

Twelve days after the booster injection all the calves are challenged with an $ID_{60}$ of $2 \times 10^8$ M. bovis EPP 63 piliated organisms. Two out of the four control calves contracted IBK whereas all the test calves were found to be protected.

EXAMPLE 4

A. M. bovis EPP 63 piliated colonies are grown on a suitable solid agar, for example Müller-Hinton Agar from BBL catalogue No. 96242. Six serial plate passages are carried out, picking four to six colonies for spreading on each passage, so as to stabilize the piliated phase. Subsequent culture was again on an agar conducive to piliated phase growth, conveniently the Müller-Hinton Agar mentioned above.

Twenty to forty colonies were then picked from the agar surface with sterile cotton swabs which were then rinsed in for example 10 cc of 1:20 trypiticase soy broth. The resulting solution was mixed until homogeneous.

Plates containing a suitable Müller-Hinton Agar, for example BBL-catalogue No. 96242 were spread with 0.1 ml of the homogeneous solution and incubated for 18-24 hours at 35°C.

The surface growth was then washed from the agar e.g. with 10 cc of the solution of trypiticase soy broth mentioned above. The fluids are collected into a sterile container. An quantity solution of 37% formalin (0.3% by volume of formalin with respect to the final vaccine composition) was added to the contents of the sterile container in order to inactivate the M. bovis organisms. The resulting mixture was mixed for 24 hours using a magnetic stirrer, by which time the wet particle size was in the range 0.01 to 0.2 mm.

Aluminium hydroxide gel containing 75% water was added as adjuvant to give a concentration of $Al_2O_3$ in the mixture of 0.5% by weight. The resulting vaccine composition was then mixed thoroughly and dispensed into sterile vaccine vials in an amount corresponding to 0.3 mg dry weight of antigen per vial.

B.  Repeat the above procedure but using <u>M. bovis</u> strain
    FLA 64.

C.  Repeat procedure A, but preparing cultures of both
    EPP 63 and FLA 64 and producing a polyvalent vaccine
    therefrom containing preferably 0.15 mg of antigen
    derived from each strain, per vial.

REFERENCE EXAMPLE

This Example was carried out to demonstrate the cross-reactivity of a trivalent M. bovis vaccine. The test was carried out on rabbits.

A vaccine containing a mixture of pure pili of M. bovis IBH 63, MED 72(4R), FLA 64 was administered to a group of four rabbits. The vaccine contained equal amounts of the different pili and was administered in an amount of 100µg per animal. The pili types were selected on the basis that they were members of strain groups consisting of more than one strain. Antiserum was obtained from the animals by standard procedures and tested by the above mentioned ELISA methods for cross-reactivity with pilus from various strains as well as the homologous strains.

The values obtained were arbitrarily nomalized with the highest level (reactivity with FLA 64 pili) being taken as 100. The results are shown in the following table and represent the average of the values obtained for all the rabbits.

| Strain group | Pilus used to test for cross reactivity with antiserum | Neutralization factor |
|---|---|---|
| I | WSE 64 (2L) | 49 |
|  | IBH 68 | 73 |
| II | EPP 63 | 18 |
| III | 1965 Glenn | 22 |
| IV | IVI 64 | 77 |
|  | MED 72 (4R) | 89 |
|  | MED 72 (19L) | 78 |
|  | WSE 64 (13R) | 78 |
| V | MAC 74 | 21 |
| VI | FLA 64 | 100 |
|  | ATC 10,900 | 99 |
|  | 8613(1) | 93 |

The above results demonstrate that the antigenicity of each pilus in the mixture was comparable with a monovalent vaccine containing the same pilus in three times the amount; and also that mixed pilus vaccines can induce antibodies to all serotypes in the vaccine.

C L A I M S

1.      A vaccine composition for cattle, capable of
raising the antibody level of cattle to a level sufficient
to provide protection against infection by one or more
strains of <u>Moraxella bovis</u> (<u>M. bovis</u>), said vaccine compri-
sing pili derived from one or more strains of <u>M. bovis</u>
capable of inducing antibodies able to cross-react with
pili of said first mentioned strain or strains to an
extent sufficient to provide protection there against, and
a pharmaceutically acceptable carrier.

2.      A vaccine as defined in Claim 1, characterised in
that it comprises pili in the substantial absence of any
other <u>M. bovis</u> cellular components.

3.      A vaccine as defined in Claim 1 or Claim 2, characterised in that the pili is derived from one or more of
the following strains of <u>M.bovis</u>:

|  | ATCC NO. |
| --- | --- |
| IBH 68 | 39222 |
| EPP 63 | 39218 |
| 1965 Glenn 5004 | 39225 |
| MED 72 (19L) | 39223 |
| MAC 74 (2554 R) | 39228 |
| FLA 64 | 39219 |
| NTN 63 | 39232 |
| GIN 63 | 39229 |

and strains having the same pilus type as the foregoing.

4.      A vaccine as defined in Claim 3, characterised in that it contains pili capable of inducing antibodies able to react and/or cross-react with pili of at least six of the strains set forth in Claim 3.

5.      A vaccine as defined in any one of the preceding claims in unit dosage form.

6.      A vaccine unit dosage form as defined in Claim 5 for administration by injection containing 3 to 5mg of pili per 3 ml of carrier, and substantially no other cellular components.

# C L A I M S

## (Applicable only to Austria)

1.      A method of preparing a vaccine composition for protecting cattle against infection by one or more strains of Moraxella bovis (M.bovis) which comprises, cultivating piliated organisms of one or more strains of M.bovis, harvesting said piliated organisms, if desired separating the pili from the cells and isolating said pili, and forming a mixture of said pili and/or piliated whole cells with a pharmaceutically acceptable carrier, wherein the pili contained in the resulting vaccine when administered to cattle is capable of inducing antibodies able to cross-react with said first mentioned strain or strains of M.bovis to an extent sufficient to provide protection against infection thereby.

2.      A method as defined in Claim 1, characterised in that said cultivation comprises cloning said organisms for the piliated phase.

3.      A method as defined in Claim 1 or 2, characterised in that pili is isolated and the resulting vaccine contains pili in the substantial absence of other cellular components.

4.      A method as defined in any one of the preceding claims, characterised by cultivating one or more of the following strains of M.bovis in the piliated phase:

|              | <u>ATCC</u> |
|--------------|------|
| IBH 68       | 39222 |
| EPP 63       | 39218 |
| 1965 Glenn 5004 | 39225 |
| MED 72 (19L) | 39223 |
| MAC 74 (2554R) | 39228 |
| FLA 64       | 39219 |
| NTN 63       | 39232 |
| GIN 63       | 39229 |

and strains having the same pilus type as the foregoing
and forming said mixture from pili or piliated whole cells
of one or more of said strains.

5.     A method as defined in Claim 4, characterised in
that the cultivated strain or strains is or are selected
such that the pili thereof is capable of inducing antibodies able to react and/or cross-react with pili of at
least six of the strains set forth in Claim 4.

6.     A method of preparing a vaccine composition in unit
dosage form which comprises preparing a vaccine composition as defined in any one of the preceding claims and
forming the composition into a unit dosage form by a known
method.

7.     A method as defined in Claim 6, for producing an
injectable unit dosage form, containing 3 to 5 mg of pili
per 3 ml of carrier, and substantially no other cellular
components.